# EUROPÄISCHE PATENTANMELDUNG

(11) **EP 0 776 635 A1**
(43) Veröffentlichungstag der Anmeldung: **04.06.1997**
(21) Anmeldenummer: 97101057.4
(22) Anmeldetag: 08.10.1993
(51) Int. Cl.: A61B 17/70, A61B 17/86

(54) **Verankerungselement**

(30) Priorität: 09.10.1992 DE 4234118
(62) Teilanmeldung aus: 93922921.7
(71) Anmelder: BIEDERMANN MOTECH GmbH, D-78054 Villingen-Schwenningen (DE)
(72) Erfinder: Harms, Jürgen, 76337 Waldbronn (DE); Shufflebarger, Harry L., Miami, Florida 33155 (US); Biedermann, Lutz, 78048 VS-Villingen (DE)
(74) Vertreter: Prüfer, Lutz H., Dipl.-Phys.

(57) **Zusammenfassung**

Es wird ein Verankerungselement mit einem zur Knochenverankerung bestimmten Schaft 2 und einem mit einer Stange 10 verbindbaren Kopf 3 mit einem im wesentlichen U-förmigen Querschnitt, der an seinem Grund 5 mit dem Schaft 2 verbunden ist, und zwei einen Kanal 4 zur Aufnahme der Stange 10 bildende freie Schenkel 6, 7 hat, geschaffen. Die freien Schenkel 6, 7 weisen ein sich in Richtung der Schenkel erstreckendes Innengewinde 8 auf. Ein Fixierelement 11 und ein die Schenkel von außen umfassendes Element 12 sind vorgesehen. Damit die Dimensionierung der Verankerung verkleinert werden kann, weisen die freien Schenkel ein Außengewinde 9 und das Element 12 ein mit diesem zusammenwirkendes Innengewinde auf. Das Fixierelement 11 weist ein mit dem Innengewinde 8 zusammenwirkendes Gewinde zum Einschrauben in dieses auf. Das Element 12 ist als beidseitig offene Mutter ausgebildet.

## Beschreibung

Die Erfindung betrifft ein Verankerungselement mit einem zur Knochenverankerung bestimmten Schaft und einem mit einer Stange verbindbaren Kopf mit einem im wesentlichen U-förmigen Querschnitt, der an seinem Grund mit dem Schaft verbunden ist und zwei einen Kanal zur Aufnahme der Stange bildende freie Schenkel hat, wobei die Schenkel ein Innengewinde und ein Außengewinde aufweisen, und mit einem die Schenkel von außen umfassenden, ein mit dem Außengewinde zusammenwirkendes Innengewinde aufweisenden Element und einem ein mit dem Innengewinde der Schenkel zusammenwirkendes Gewinde aufweisenden Fixierelement.

Ein derartiges Verankerungselement ist aus der EP-A-0487830 bekannt. Die Stange weist einen dreieckförmigen Querschnitt auf und dient zur Verspannung gegen Torsionsbewegung. Zur eigentlichen Längsstabilisierung sind Platten vorgesehen, die der vorzunehmenden Formkorrektur entsprechend gebogen werden. Die Platten beanspruchen naturgemäß verhältnismäßig viel Platz.

Aufgabe der Erfindung ist es, das Verankerungselement so auszubilden, daß die Dimensionierung der Verankerung verkleinert werden kann. Das spielt insbesondere deshalb eine Rolle, weil derartige Verankerungselemente mit den damit zu verbindenden Stangen als Bestandteil von Korrekturimplantaten für die menschliche Wirbelsäule verwendet werden und deshalb kleine Dimensionen anzustreben sind. Diese Aufgabe wird durch das Verankerungselement nach Anspruch 1 gelöst.

Weitere Merkmale und Zweckmäßigkeiten der Erfindung ergeben sich aus der Beschreibung von Ausführungsbeispielen anhand der Figuren.

Von den Figuren zeigen:
- Fig. 1: eine Seitenansicht einer ersten Ausführungsform des Verankerungselementes;
- Fig. 2: eine Frontansicht auf die in Fig. 1 gezeigte Vorrichtung;
- Fig. 3: einen Schnitt entlang der Linie III - III in Fig. 1;
- Fig. 4: einen Schnitt entlang der Linie IV - IV in Fig. 2;
- Fig. 5: eine Seitenansicht einer zweiten Ausführungsform;
- Fig. 6: einen Schnitt entlang der Symmetrieebene einer abgewandelten Ausführungsform in Explosionsdarstellung;
- Fig. 8: die Darstellung gemäß Fig. 7 mit fixierter gerader Stange;
- Fig. 9: dieselbe Ausführungsform mit konvex gebogener Stange; und
- Fig. 10: dieselbe Ausführungsform mit konkav gebogener Stange.

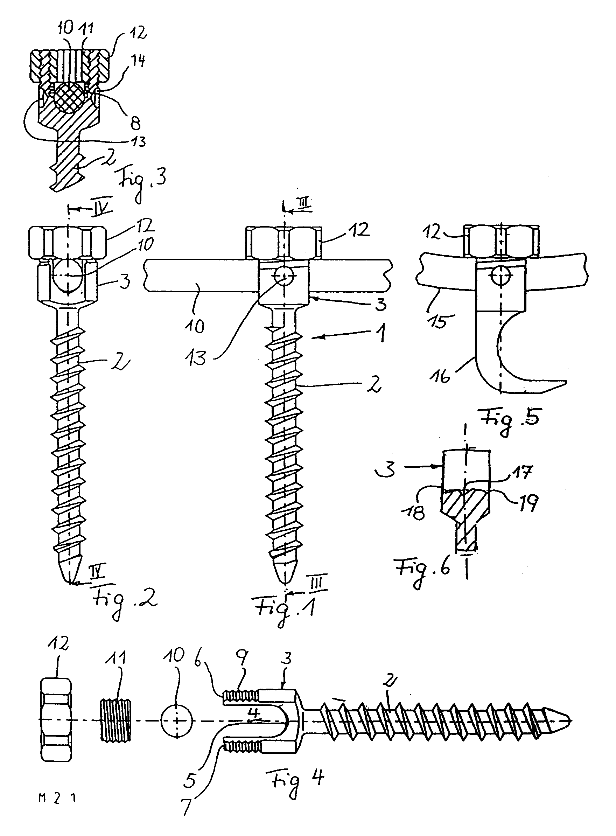

Das in Fig. 1 gezeigt Verankerungselement umfaßt eine eigentlich in eine Wirbelsäule einzubringende Schraube 1 mit einem Gewindeschaft 2 und einem Kopf 3. Der Kopf 3 weist eine zur Mittenachse des Gewindeschaftes 2 symmetrisch angeordnete U-förmige Ausnehmung 4 auf, deren Grund 5 zu dem Gewindeschaft 2 hin gerichtet ist. Die Seitenwandung des die U-förmige Ausnehmung 4 begrenzenden Kopfes wird durch freie Schenkel 6, 7 gebildet. Wie am besten aus den Fig. 3, 4, und 5 zu ersehen ist, ist im Inneren des Kanales eine konzentrisch zur Mittenachse des Gewindeschaftes 2 ausgebildete Bohrung mit einem Innengewinde 8 vorgesehen. Der Kopf 3 selbst ist zylindrisch ausgebildet und weist, wie am besten aus Fig. 4 ersichtlich ist, im Bereich der freien Schenkel 6, 7 ein Außengewinde 9 auf.

Damit mit dieser Schraube eine Stange verankert werden kann, ist ein als Gewindebolzen ausgebildetes Fixierelement 11 vorgesehen. Das Fixierelement 11 weist ein mit dem Innengewinde 8 zusammenwirkendes Außengewinde zum Einschrauben in die U-förmige Ausnehmung 4 auf. Auf seiner dem Grund 5 abgewandten Fläche weist das Fixierelement einen Schlitz oder eine entsprechend andere Ausnehmung zum Eingreifen mittels eines Schraubendrehers auf. Ferner ist ein die beiden U-förmigen Schenkel 6, 7 von außen umfassendes Element in Form einer Überwurfmutter 12 vorgesehen, deren Gewinde mit dem Außengewinde 9 zusammenwirkt. Die Drehrichtung des Innengewindes 8 und des zugehörigen Fixierelementes 11 einerseits und des Außengewindes 9 und der Überwurfmutter 12 andererseits ist entgegengesetzt gerichtet. Wie am besten aus Fig. 3 ersichtlich ist, weist der Grund 5 der U-förmigen Ausnehmung einen Radius auf, der nur soviel größer als der Radius der aufzunehmenden Stange 10 ist, daß die Stange in die U-förmige Ausnehmung leicht einsetzbar bzw. leicht aus dieser herausnehmbar ist. Das Innengewinde 8 und das Außengewinde 9 erstrecken sich jeweils so weit nach unten, also in Richtung des Grundes 5, daß die Projetion auf die Symmetrieachse einen Abstand vom Grund 5 aufweist, der kleiner ist als der Durchmesser der aufzunehmenden Stange 10.

Bei der in Fig. 5 gezeigten Ausführungsform ist der Kopf nicht mit einem Gewindeschaft, sondern mit einem als Haken 16 ausgebildeten Schaft verbunden. Dieser dient in gleicher Weise zur Verbindung mit einem Wirbelsäulenelement, wobei der Haken in die Wirbelbogen eingebracht wird.

Bei der in Fig. 6 beschriebenen Ausführungsform weist der Grund der U-förmigen Ausnehmung eine zu der Mittenachse des Innengewindes 8 konzentrische Ausnehmung 17 in Form einer Senkbohrung auf. Die beiden äußeren Ränder 18, 19 des Grundes sind nach außen zum Schaft hin abfallend gewölbt. Dadurch wird erreicht, daß beim Aufnehmen einer gebogenen Stange nicht nur eine punktförmige Auflage auf dem Grund der U-förmigen Ausnehmung 4, sondern eine größere Auflagefläche gebildet wird. Die Wölbung zum Schaft hin dient dazu, bei einer Stange, die zum Schaft hin gebogen ist, einen besseren Formschluß zu gewährleisten.

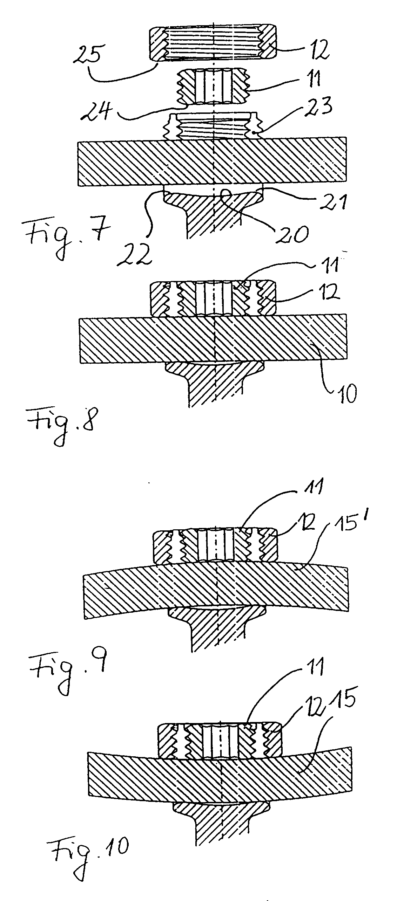

In den Fig. 7 bis 10 ist jeweils der Kopf einer weiteren Ausführungsform mit einer damit zu verbindenden Stange gezeigt. Der mit dem Kopf verbundene Schaft kann sowohl als Gewindeschaft 2 als auch als Haken 16 ausgebildet sein.

Wie aus den Figuren ersichtlich ist, ist der Grund der jeweiligen U-förmigen Ausnehmung gewölbt ausgebildet. Die Wölbung weist einen inneren konkaven Abschnitt 20 und zwei symmetrisch zur Mittenachse der Schraube angeordnete nach oben erhaben hervorstehende gewölbte Bereiche 21, 22 auf. Der Abstand der jeweils höchsten Stelle der wulstförmigen Erhebung 21 bzw. 22 von der Mittenachse der Schraube ist im wesentlichen gleich dem Abstand der Mitte 23 zwischen dem Radius des Innengewindes 8 und dem Radius des Außengewindes 9 von der Symmetrieachse der Schraube.

Wie am besten aus den Fig. 1, 3 und 5 entnehmbar ist, weist der Kopf 3 zwei gegenseitig um 180° versetzte Ausnehmungen 13, 14 auf, die im wesentlichen jeweils um 90° gegen die U-förmige Ausnehmung versetzt sind und die zum Eingreifen mittels eines Handhabungswerkzeuges, beispielsweise einer Greifzange dienen.

Im Betrieb wird zunächst die Schraube 1 in die Wirbelsäule eingeschraubt. Anschließend wird die Stange 10 bzw. 15, 15' in die U-förmige Ausnehmung 4 eingelegt und durch Einschrauben des Fixierelementes 11 in das Gewinde 8 fixiert. Dann wird die Überwurfmutter 12 aufgeschraubt. Das Fixierelement und die Überwurfmutter 12 werden nun getrennt jeweils so weit in Richtung des Grundes 5 gedreht, bis jedes der beiden Teile eine gewünschte Haltekraft auf die Stange 10, 15, 15' ausübt. Dadurch wird der Vorteil erreicht, daß die Fixierung von Fixierelement 11 und Überwurfmutter 12 relativ zu der Stange unabhängig voneinander einstellbar sind. Darüber hinaus erfolgt eine endgültige Fixation und eine Schraubensicherung durch Verklemmung.

Durch die gewölbte Ausbildung des Grundes der U-förmigen Ausnehmung und insbesondere durch die in den Fig. 7 bis 10 gezeigte Ausbildung mit den einen Abstand voneinander aufweisenden erhabenen gewölbten Auflagebereichen und deren oben beschriebene Lage wird erreicht, daß jede zu fixierende Stange, egal, ob sie als gerade Stange 10 oder als konvex oder konkav gebogene Stange 15, 15' ausgebildet ist, auf zwei flächenhaft ausgebildeten Bereichen 21, 22 aufliegt. Wie am besten aus Fig. 7 ersichtlich ist, sind zusätzlich die der Stange zugewandten unteren Ränder 24, 25 des Fixierelementes 11 und der Überwurfmutter 12 abgerundet beziehungsweise konvex gewölbt. Dadurch wird erreicht, daß alle Angriffsstellen, die bei der Fixierung auf die zu ergreifende Stange einwirken, ohne scharfe Ränder an die Stange angreifen. Darüber hinaus wird durch die beschriebene Lage erreicht, daß die Angriffsbereiche der beiden erhabenen Stellen 21, 22 des Grundes gerade zwischen den beiden Angriffsstellen des Fixierelementes 11 und der Überwurfmutter 12 liegen. Dadurch wird eine besonders gute Fixierung und eine besonders geringe Streßbelastung erreicht. Wie am besten aus den Fig. 8 bis 10 ersichtlich ist, wird die gute Fixierung für jede Art der Biegung der Stangen 10, 15, 15' erreicht. Bei den oben beschriebenen Ausführungsbeispielen ist der Kopf 3 jeweils mit dem ein Gewinde aufweisenden Schaft beziehungsweise dem hakenförmigen Schaft fest verbunden. Nach einer abgewandelten Ausführungsform sind der jeweilige Schaft und der Kopf gelenkig miteinander verbunden.

## Patentansprüche

1. Verankerungselement mit einem zur Knochenverankerung bestimmten Schaft (2, 16) und einem mit einer Stange (10, 15) verbindbaren Kopf (3) mit einem im wesentlichen U-förmigen Querschnitt, der an seinem Grund (5) mit dem Schaft (2, 16) verbunden ist und zwei einen Kanal zur Aufnahme der Stange (10, 15) bildende freie Schenkel (6, 7) hat,
wobei die Schenkel (6, 7) ein Innengewinde (8) und ein Außengewinde (9) aufweisen,
und mit einem die Schenkel (6, 7) von außen umfassenden, ein mit dem Außengewinde zusammenwirkendes Innengewinde aufweisenden Element (12) und einem ein mit dem Innengewinde (8) der Schenkel zusammenwirkendes Gewinde aufweisenden Fixierelement (11),
dadurch gekennzeichnet, daß
das Element (12) als beidseitig offene Mutter ausgebildet ist.
